Europäisches Patentamt

European Patent Office

Office européen des brevets

(11) Veröffentlichungsnummer: **0 178 372 B1**

## (12) EUROPÄISCHE PATENTSCHRIFT

(45) Veröffentlichungstag der Patentschrift: **11.09.91**

(51) Int. Cl.⁵: **D04H 1/66, A61B 19/08**

(21) Anmeldenummer: **85103757.2**

(22) Anmeldetag: **28.03.85**

(54) **Mikroporöser Mehrschichtvliesstoff für medizinische Anwendungszwecke und Verfahren zu dessen Herstellung.**

(30) Priorität: **10.10.84 DE 3437183**

(43) Veröffentlichungstag der Anmeldung:
**23.04.86 Patentblatt 86/17**

(45) Bekanntmachung des Hinweises auf die
Patenterteilung:
**11.09.91 Patentblatt 91/37**

(84) Benannte Vertragsstaaten:
**DE FR GB NL SE**

(56) Entgegenhaltungen:
**EP-A- 0 006 264**
**FR-A- 2 257 719**
**GB-A- 1 071 191**
**US-A- 3 873 486**
**US-A- 4 041 203**

(73) Patentinhaber: **Firma Carl Freudenberg**
**Höhnerweg 2-4**
**W-6940 Weinheim/Bergstrasse(DE)**

(72) Erfinder: **Groitzsch, Dieter, Dr.**
**Hermann-Löns-Strasse 6A**
**W-6945 Hirschberg 2(DE)**
Erfinder: **Fahrbach, Erich, Dr.**
**Zinkgräfstrasse 62**
**W-6940 Weinheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen mikroporösen Mehrschichtvliesstoff für medizinische Anwendungszwecke, z.B. als OP-Einwegmaterial, mit den Merkmalen des Oberbegriffs des Patentanspruchs 1. Es wird weiterhin ein Verfahren zur Herstellung eines solchen Kompositvliesstoffes vorgeschlagen.

Gattungsgemäße Mehrschicht- bzw. Kompositvliesstoffe sind für medizinische Anwendungszwecke und auch als OP-Einwegmaterialien bekannt. Die Mikrofaserschicht aus hydrophoben Fasern dient als Filtermedium für feinste Partikel und Bakterien. Zur Verhinderung des Durchtrittes der feinen Fasern an die Oberfläche sowie des auszufilternden Materials ist die Mikrofaserschicht auf beiden Seiten durch Hüllvliesstoffe abgedeckt. Auf diese Weise wird auch der Bakteriendurchtritt unterbunden. Es ist eine weitgehend undurchlässige wasserdichte Konstruktion des Laminats notwendig.

EP-A-0 006 264 beschreibt einen Komposit-Vliesstoff für medizinische Anwendungszwecke, der mindestens zwei hydrophobe Mikrofaserlagen enthält mit Faserdurchmessern bis zu 10 $\mu$m. Jede Lage besitzt ein Basisgewicht von 10 g/m² und eine Anfangsdichte bis zu 0,15 g/m . Ferner vorhanden ist mindestens eine Abdeckschicht aus luftdurchlässigem Vliesstoff. Alle Schichten sind an ihren Kanten miteinander verbunden. Die Vliesstoffe können bestehen aus zerfasertem Film, aus Spinnvlies- oder Naß- oder Trockenvliesstoff sowie auch aus kardierten Fasern. Besonderes Gewicht wird auf die Stabilität der einzelnen Schichten gelegt, welche insbesondere dadurch gesteigert werden kann, daß der Kompositvliesstoff eine Verstärkungsschicht aus hydrophoben Fasern enthält. Es wird Wert darauf gelegt, daß die gesamte Komposit-Struktur nur minimal gebunden ist, um die Luftdurchlässigkeit und Widerstandsfähigkeit gegen Flüssigkeits- und Bakteriendurchtritt nicht negativ zu beeinflussen. Dies wird durch Verbinden der Schichten nur an deren Kanten erreicht. Ferner darf deswegen die Zahl der Mikrofaserschichten den Wert 4 oder 5 nicht übersteigen.

Bekannt sind OP-Materialien, OP-Kittel und OP-Abdeckstoffe, die mit hydrophoben Vliesstoffen, z.B. auf der Basis von Polypropylen oder mit Folien, kaschiert sind (DE 33 21 893 A1). Die einzelnen Schichten des Laminat-Aufbaus sind oft schlecht miteinander verbunden, so daß solche Produkte in großflächigen Anwendungsbereichen nur bedingt eingesetzt werden können. Bei Kleinteilen, z.b. chirurgischen Gesichtsmasken, wird das Laminat durch Nähen bzw. Schweißen des Dreischichtgebildes zusammengehalten. Es sind auch thermisch verschweißte Vliesstoffschichten aus synthetischen Fasern bekannt.

Alle diese Materialien vermitteln ein unangenehmes Tragegefühl, weil sie aufgrund der sehr niedrigen Luftdurchlässigkeit und des fehlenden Feuchtehaushaltes zum Schwitzen des Trägers führen.

Die Mikrofaserschicht kann in unterschiedlicher Weise hergestellt werden, z.B. durch das elektrodynamische Erspinnen von dielektrischen Polymerfasern aus leichtflüchtigen Lösungsmitteln oder das elektrostatische Erspinnen aus der Schmelze oder das Verblasen einer Schmelze. Es hat sich als nachteilig erwiesen, daß z.B. nach dem elektrostatischen Spinnverfahren aus niedrig siedenden Lösungsmitteln, wie Methylenchlorid, nur sehr harte und spröde Polymere, wie Polycarbonat, Polysulfon, Cellulosetriacetat und Polystyrol bzw. Verschnitte untereinander, verarbeitet werden können. Die Festigkeit innerhalb einer solchen Mikrofaserschicht ist sehr niedrig. Hier ist eine zumindest einseitige Abdeckung mit reißfesten Trägermaterialien unumgänglich. Beim thermischen Verschweißen solcher Laminate erhält man aber sehr harte und spröde Schmelzstellen, die zu unangenehmen Trageeigenschaften führen. Durch die Versprödung besteht zuzüglich die Gefahr einer Zerstörung und Rißbildung der Mikrofaserschicht bei mechanischer Beanspruchung.

Die bekannten Mehrschichtvliesstoffe weisen außerdem den schwerwiegenden Nachteil auf, daß sie nicht drapierfähig sind. Die mit der Versprödung verbundene Kurzdehnigkeit und das geringe Arbeitsaufnahmevermögen des Verbundstoffes führt dazu, daß Bewegungen oder mechanische Einflüsse während der Anwendung, z.B. als Schutzkleidung, zur Zerstörung der Filterschicht führen können. Bei thermisch verschweißten Materialien geht durch die thermische Behandlung der größte Teil der auf Elektrostatik bzw. Elektretfaser beruhenden Filterleistung verloren. Bei der Verwendung für OP-Kittel und OP-Abdeckmaterialien ist auch das Abbrechen, Abtragen oder Eindringen von Mikrofasern nicht erlaubt.

Die Abdichtung der Mikrofaserschicht mit einer Folienkaschierung gewährt zwar einen absoluten Schutz gegen Bakteriendurchtritt, der Tragekomfort ist jedoch außerordentlich schlecht. Bei längeren Operationen ist mit einem Hitzestau zu rechnen.

Der Erfindung liegt nun die Aufgabe zugrunde, einen mikroporösen Mehrschichtvliesstoff für medizinische Anwendungszwecke, insbesondere für den OP-Gebrauch, zu entwickeln, wobei die Produkte weich und drapierfähig sein sollen und trotzdem den beim Gebrauch auftretenden mechanischen Beanspruchungen gewachsen sein sollen. Das Material muß deshalb wasserdicht, atmungsaktiv, d.h. luftdurchlässig und wasserdampfdurchlässig sein. Der Laminataufbau soll sich auch bei mechanischen Belastungen nicht trennen. Die Filterwirkung der Mikrofaserschicht darf beim Gebrauch nicht nachlassen. Es muß weiterhin vermieden werden, daß einzelne Mikrofasern den Abdeckvliesstoff des Laminats nach außenhin durchdringen. Die erfindungsgemäße Aufgabe liegt weiterhin in der Entwicklung eines Verfahrens zur Herstellung eines Mehrschichtvliesstoffes mit den vorstehend angegebenen Eigenschaften.

Die Aufgabe wird gelöst durch das im Patentanspruch 1 gekennzeichnete Material und dessen Herstellungsverfahren gemäß Patentanspruch 7.

Der erfindungsgemäß vorgeschlagene Laminataufbau besteht in einem sehr drapierfähigen Trägermaterial aus Vliesstoff, auf dem die Mikrofaserschicht angeordnet ist. Zweckmäßig wird die Mikrofaserschicht elektrostatisch aufgesponnen. Bevorzugt wird hierbei das in DE-PS 20 32 072 beschriebene Verfahren. Die Mikrofaserschicht ist mit einem zweiten sehr drapierfähigen Vliesstoff abgedeckt.

Die Vliesstofflaminate, sowohl der Träger- als auch der Abdeckvliesstoff, sind trocken- oder naßgelegte Vliesstoffe. Bei besonderer Beanspruchung werden Spinnvliesstoffe bevorzugt. Zweckmäßig weisen die Vliesstoffe ein Gewicht von etwa 10 bis 40 g/m² auf.

Falls eine extreme Drapierfähigkeit verlangt wird, sind bindemittelfrei gebundene und leichte Stapelfaservliesstoffe vorteilhaft. Die Abriebbeständigkeit läßt sich durch bindemittelangereicherte Vliesstoffe erhöhen. Es wird vielfach bevorzugt, wenn das Mikorfasermedium beidseitig mit gleichen Materialien abgedeckt ist.

Die Abdeckvliesstoffe sind gewöhnlich hydrophobiert. Wenigstens einer der beiden Vliesstoffe kann nach einer zweckmäßigen Ausgestaltung aus hydrophilen Fasern bestehen, und nicht hydrophobiert sein. Die Druckpaste ist hydrophob und durchdringt das gesamte Dreierlaminat in Form von säulenförmigen Gebilden. Die Grundfläche dieser Gebilde kann beliebig gestaltet sein. Die Druckpaste wird somit "durchgenietet" und ermöglicht einen dauerhaften Verbund des Laminats. Für relativ dünne Laminate genügt ein einseitiger Aufdruck der hydrophoben Druckpaste für Erreichung einer ausreichenden Festigkeit. In Fällen, wo das Laminat eine größere Dicke hat, empfiehlt sich für das Aufbringen der Druckpaste ein beidseitiger Aufdruck. Dieser ist so vorzunehmen, daß sich die jeweils eingepreßten Druckpastenmengen einander im Inneren des Laminates berühren. Dies kann durch spiegelbildliche Aufbringung der beiderseitigen Muster erreicht werden, technisch machbar indessen unter beiderseitiger Verwendung von Musterungen, die durch in einem Winkel zueinander versetzbare Stäbchen gebildet werden. Eine Winkelzuordnung der Stäbchen von 90° hat sich besonders bewährt. Eine gegenseitige Überschneidung und damit Berührung ist in diesem Fall immer gewährleistet. Die Druckpaste wird zweckmäßig als wässrige Pastenemulsion aufgetragen. Der fertige Druck ist in dem gewünschten Maße elastisch.

Das Verfahren zur Herstellung des Mehrschichtvliesstoffes besteht darin, daß die Mikrofaserschicht zunächst auf den sehr drapierfähigen Vliesstoff aufgetragen wird, wobei sich insbesondere das elektrostatische Aufspinnen bewährt hat. Es wird dann der Abdeckvliesstoff zugeführt und das dreischichtige Laminat vor dem Aufwickeln leicht angedrückt. Das Laminat wird dann bei Temperaturen zweckmäßig oberhalb 60°C in Wasser gewaschen, abgequetscht und dann durch eine hydrophobiermittelhaltige Flotte geführt, nochmals abgequetscht und mit der hydrophob eingestellten elastischen Bindemittelpaste bedruckt. Das bedruckte Material wird dann getrocknet.

Das Waschen des Laminats vor der Hydrophobierung dient der Entfernung von unerwünschten Fremdstoffen, z. B. Spinnpräparationen der Fasern bzw. Emulgatoren bei bindemittelgebundenen Hüllvliesstoffen. Es werden weiterhin etwa vorhandene Schaumhilfsmittel, Netzmittel oder dergl. weitgehend entfernt. Das Waschwasser soll wenigstens 60°C heiß sein, damit sichergestellt ist, daß das Mikrofasermedium gut durchnetzt wird.

Dieses gewaschene Material wird dann möglichst auf minimale Restfeuchte = Naßaufnahme abgequetscht und der Hydrophobierflotte zugeführt. Die Restfeuchte, bezogen auf das Trockengewicht des Laminates ist abhängig vom Gewicht, Dicke und Aufbau des Laminates sowie den Abquetschbedingungen. Damit aus dem anschließend passierten Tränkbad in die Hydrophobiermittelflotte aufgenommen werden kann, ist es notwendig, daß nach dem Waschen stärker abgepreßt wird als nach der Hydrophobiermitteltränkung.

Es gelten dabei folgende mathematische Bezeichnungen:

$$n_2 = \frac{G_2\,(n_1 + 1)}{G_1} - 1;$$

$$\Delta g = (n_2 - n_1)\cdot\frac{F}{100}\cdot g_{HM} \qquad n_1 = \frac{N_1}{100}$$

$$n_2 = \frac{N_2}{100}$$

$$g_{FM} = g_{HM} + \Delta G;$$

Hierin bedeuten:

$g_{HM}$...     Trockengewicht des unausgerüsteten Halbmaterials in g/m²
$g_{FM}$...     Trockengewicht des ausgerüsteten Fertigmaterials in g/m²
$\Delta g$...     Gewicht der Ausrüstung in g/m²
$G_1$...     Gewicht des feuchten Halbmaterials nach dem Abquetschen des Waschwassers in g/m².
$G_2$...     Gewicht des feuchten Halbmaterials nach dem Abquetschen der 2. Trankung (Naß-in-Naß-Tränkung)
$n_2$...     Naßaufnahme (= Restfeuchte) nach dem Abquetschen der 2. Tränkung bezogen auf Halbmaterialgewicht $g_{HM}$
$n_1$...     Naßaufnahme nach dem Abquetschen des Waschwassers bezogen auf Halbmaterialgewicht $g_{HM}$
$N_2$...     wie $n_2$, jedoch in % ausgedrückt
$N_1$...     wie $n_1$, jedoch in % ausgedrückt

Daraus geht deutlich hervor, daß $n_2$ größer als $n_1$ sein muß. ($n_2 > n_1$). Im Regelfall sollten sich die Werte für ($n_2$ - $n_1$) bzw. $n_1$ in folgenden Grenzen bewegen:

$n_2$ - $n_1 \geqq 0{,}05$ ($N_2$ - $N_1 \geqq 50$ %)
$n_1 \leqq 2{.}0$ ($N_1 \leqq 200$ %)

wobei es aus trocknungsenergetischen Gründen vorteilhaft ist $n_1$ so tief wie möglich zu halten, d. h. nach dem Waschen so stark wie möglich, ohne Schädigung des Materials abzuquetschen.

Eine Zwischentrocknung ist nicht notwendig. Das Material wird dann bedruckt. Es war überraschend, daß die Druckpaste ohne Schwierigkeit die extrem feine Mikrofaserschicht durchdringt und unter Ausbildung stäbchenförmiger Stege einen festen Verbund gewährleistet. Es wurde überraschenderweise gefunden, daß durch das Bedrucken des noch feuchten Materials die Druckpaste problemlos die Mikrofaserschicht durchdringt, wobei diese jedoch nicht zu voluminös und schwer sein darf. Das Gewicht der Mikrofaserschicht muß sich deshalb innerhalb des Bereichs von 0,5 bis 60 g/m² bewegen.

Die Druckpaste besteht im wesentlichen aus einer emulgatorarmen Polymerdispersion, die ausschließlich hydrophobe Bestandteile, insbesonere hydrophobe Monomere, enthält, sowie ein körperarmes hochmolekulares Verdickungsmittel und ein Hydrophobiermittel. Eine derartige sehr emulgatorarme Druckpaste, die keine oder nur Spuren an wasserlöslichen Agentien aufweist, und zusätzlich ein Hydrophobiermittel enthält, gewährleistet das einwandfreie Durchdringen des Mikrofiltermediums und verhindert eine Leckage gegenüber Wasser oder eine Versehlechterung der Wasserdichtigkeit des hergestellten Dreischichtgebildes.

Das Druckmuster wird zweckmäßig als Schablonendruck aufgetragen, wobei die Anordnung der Druckstellen weitgehend beliebig ist. Bei sehr spröden Polymeren der Mikrofaserschicht, wie sie beispielsweise nach dem elektrostatischen Spinnen aus einer Lösung vorliegen, ist es zweckmäßig, wenn der Abstand der elastischen Druckbindepunkte eng gesetzt wird.

Das Zusammenlaminieren der drei Schichten mit der beschriebenen Druckpastendurchnietung verleiht dem Material eine hohe Reißfestigkeit, Spaltfestigkeit sowie Flexibilität, selbst bei sehr harten und spröden Polymermaterialien in der Mikrofaserschicht. Je nach Wahl des Mikrofasermaterials, der Komponenten in der Druckpaste sowie des Hydrophobiermittels in der Druckpaste und in der Tränkung kann der Mehrschichtvliesstoff den Wünschen des Verbrauchers entsprechend eingestellt werden. Es ist sowohl möglich Einwegartikel (Disposables) auch mehrfach verwendbare Materialien herzustellen. Für eine Waschbestän-

4

digkeit und Reinigungsbeständigkeit ist es wichtig, vernetzbare Hydrophobiermittel und Bindemittel zu verwenden. Derartige Mittel und ihre entsprechenden Eigenschaften sind bekannt und als Handelsprodukte erhältlich.

Wenn voluminöse und schwerere Materialien mit der Druckpaste durchgenietet werden sollen ist es zweckmäßig, der Hydrophobierflotte wirksame Entschäumer, z. B. auf Silikonbasis, zuzusetzen. Die Druckpaste wird dann zweckmäßig in aufgeschäumter Form eingesetzt. Durch die Berührung der Schaumpaste mit dem entschäumerhaltigen noch feuchten Kompositvliesstoff erfolgt dann während des Druckvorganges eine spontane Entschäumung unter starker Herabsetzung der Viskosität der Druckpaste. Hierdurch wird das Ein- bzw. Durchdringen auch dickerer und voluminöserer Kompositvliesstoffe erleichtert.

Die Wasserdichtheit ergibt sich durch das Bedrucken des hydrophobiermittelgetränkten Materials mit der elastischen hydrophoben Druckpaste. Der Unterschied zwischen nicht mit Hydrophobiermittel und mit Hydrophobiermittel getränktem 3-Schichtlaminat ist um so ausgeprägter, je höher das Gewichtsverhältnis der Abdeckvliesstoffe zu der Mikrofaserschicht ist. Bei schwereren Kompositen empfiehlt sich ein beidseitiger Druckauftrag. Die Druckpunkte können dabei genau übereinander bzw. spiegelbildlich zueinander gesetzt werden. Bewährt hat sich ein stäbchenförmiges Druckmuster, wobei die sehr schlanken Stäbchen dann nicht mehr exakt spiegelbildlich sondern in einem Winkel zueinander, zweckmäßig im Winkel von 90°, aufgetragen werden, weil sie sich an den Kreuzungsstellen berühren und dadurch die Laminatfestigkeit gewährleisten.

Die zur Abdeckung verwendeten Vliesstoffe sind in der Regel hydrophobiert. Zur Erhöhung der Wasseraufnahmefähigkeit kann wenigstens eines der Laminate aus zellwoll- oder zellstoffhaltigen Vliesstoffen bestehen. Derartige Vliesstoffe sind aufgrund ihrer hohen Wasseraufnahme und ihrer ungekräuselten Struktur relativ flach. Sie lassen sich deshalb leicht von der Druckpaste durchdringen. Derartige Vliesstoffe sind wegen des hohen Feuchtehaushaltes angenehm im Tragen und immer dann bevorzugt, wenn ein erhöhter Tragekomfort gewünscht wird.

Durch einen mechanischen Weichmachungsprozeß kann die Drapierfähigkeit der zur Abdeckung verwendeten Vliesstofflaminate erhöht werden. Dies ist besonders dann vorteilhaft, wenn das Mikrofasermedium von naß gelegten Hüllvliesstoffen umgeben ist. Die Hydrophobierung des Hüllvliesstoffes kann gegebenenfalls entfallen, wenn eine oder zwei saugende Oberflächen des dreischichtigen Mehrvliesstoffes erwünscht sind. In diesem Falle sind Vliesstoffe mit einem sehr hohen Anteil an Zellwolle und/oder Zellstoff zweckmäßig, die dann nicht vollflächig sondern nur teilflächig gebunden sind. Nach dem Waschen kommt die Saugkraft der Fasern dann voll zum Tragen. Im Falle der Verwendung von quellfähigen Bindemitteln, d. h. von solchen Bindemitteln die nicht saugen aufgrund des Zusatzes von grenzflächenaktiven Substanzen (Tensiden), sondern aufgrund ihres Polymeraufbaues und geringen Vernetzungsgrades, ist auch eine ganzflächige Bindung der Vliesstoffe möglich. Ein Auswaschen der wasserlöslichen Bestandteile führt hier nicht zu einem Verlust an Saugkraft. Die Mikrofaserschicht besteht aus Fasern mit einem Durchmesser zwischen 0,1 und 10 $\mu$m. Die Auflagestärke ist abhängig von dem späteren Verwendungszweck und bewegt sich innerhalb des Bereichs von 0,5 bis 60 g/m$^2$. Speziell beim elektrostatischen Erspinnen aus Lösung können Gewichte von weniger als 1 g/m$^2$ Mikrofasern mit extrem gleichmäßiger Verteilung erzielt werden. Der bevorzugte Bereich des Mikrofaserauftrages liegt in der Regel zwischen 1 und 30 g/m$^2$.

In Abhängigkeit von den späteren Anforderungen an Wasserdichtheit, Bakteriendichtheit, Wasserdampfdurchlässigkeit und der erwarteten Filterleistung wird die Auflage der Mikrofasern bestimmt. Für eine Wasserdichtheit von 40 mbar, gemessen nach DIN-Norm 53 886/77, genügt z. B. eine Mikrofaserauflage von 8 g/m$^2$ aus 93 Gew.-% Polycarbonat und 7 Gew.-% Polystyrol elektrostatisch aus einer Lösung ersponnener Mikrofasern einer durchschnittlichen Feinheit von 4,5 $\mu$m, wenn die Mikrofaserschicht beidseitig mit 20 g/m$^2$ schweren zellstoff-zellwollhaltigen Naßvliesen abgedeckt wird. Ein derartiges Dreischichtgebilde ist in Beispiel 2 beschrieben.

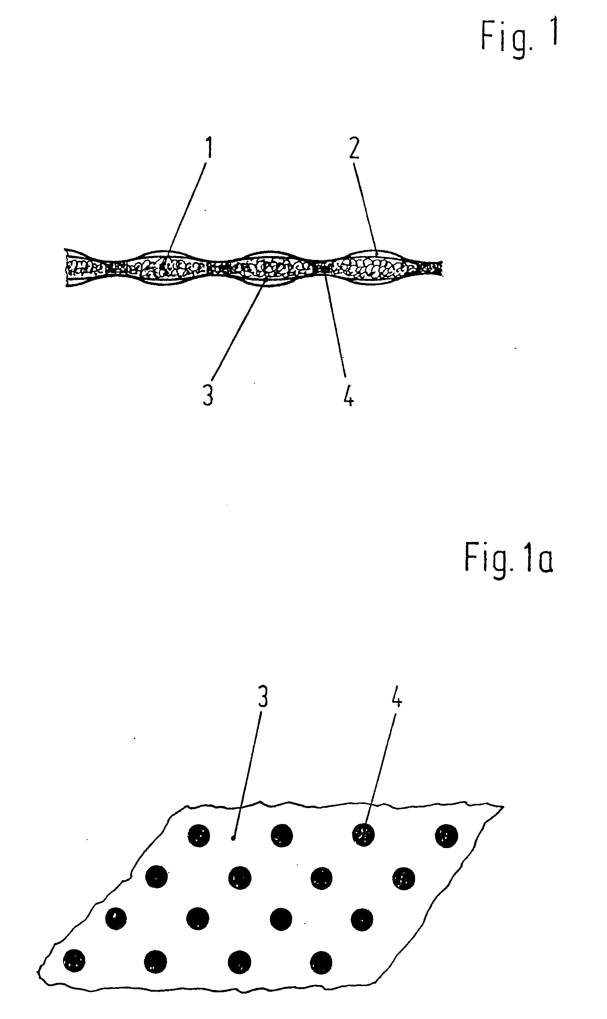

Figur 1 bis 6 zeigen den Aufbau des Mehrschichtvliesstoffes und ein Verfahrenschema.

Figur 1 zeigt einen Querschnitt durch den dreilagigen Mehrschichtvliesstoff. Die Mikrofaserschicht 1 befindet sich auf dem hydrophoben Trägervliesstoff 2 und ist mit dem ebenfalls hydrophoben Abdeckvliesstoff 3 versehen. Die hydrophobe elastische Druckpastendurchnietung 4 gewährleistet einen festen Verbund.

Figur 1a zeigt eine Draufsicht auf einen Mehrschichtvliesstoff gemäß Figur 1. Die Druckpunkte 4 sind musterförmig auf dem wasserabweisenden Abdeckvliesstoff 3 angeordnet.

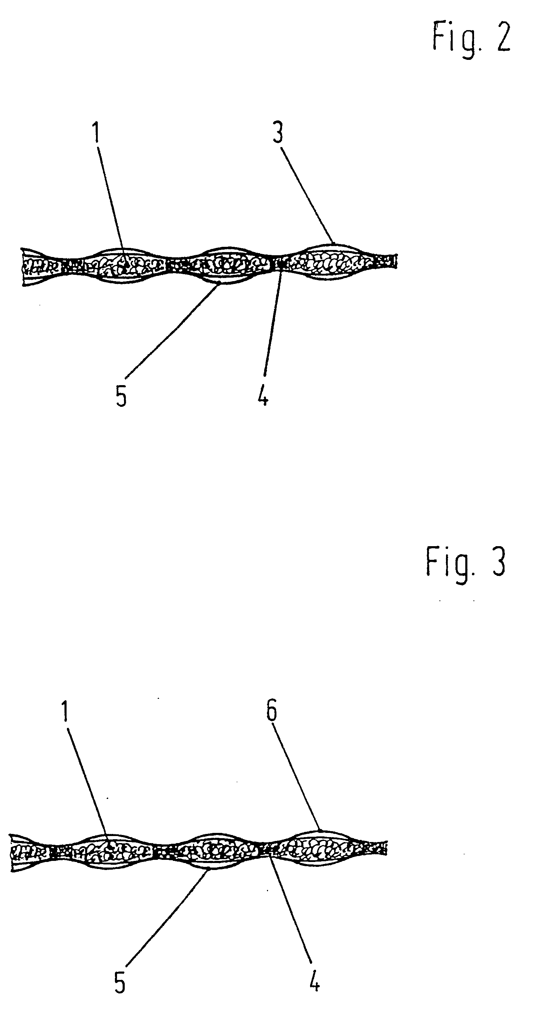

Figur 2 zeigt einen dreilagigen Mehrschichtvliesstoff, bei dem das dielektrische Mikrofasermedium 1 auf einem saugenden Trägervliesstoff 5 angeordnet und mit einem wasserabweisend ausgerüsteten Abdeckvliesstoff 3 versehen ist. Die hydrophobe elastische Durchnietung 4 gewährleistet den Verbund. Figur 3 zeigt einen beidseitig saugenden dreilagigen Mehrschichtvliesstoff. Das dielektrische Mikrofasermedium 1 befindet sich auf einem saugenden Trägervliesstoff 5 und ist mit dem ebenfalls saugenden Abdeckvliesstoff 6 versehen. Die hydrophobe elastische Druckpastendurchnietung 4 gewährleistet den Verbund.

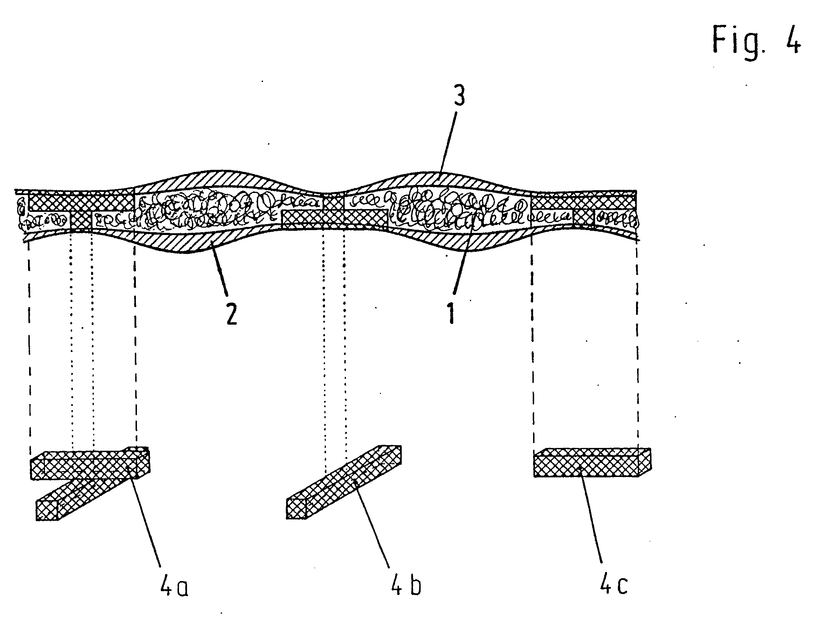

Figur 4 verdeutlicht die Funktion der hydrophoben elastischen Druckpastendurchnietung 4. Das dielektrische Mikrofasermedium 1 ist von den Hüllvliesstoffen 2 und 3 umgeben. Die Druckpastenpunkte 4 sind unterschiedlich angeordnet. Es befinden sich nebeneinander ein sich überkreuzender Binderdruck 4a, ein unten aufgetragener Binderdruck 4b und ein oben aufgetragener Binderdurck 4c.

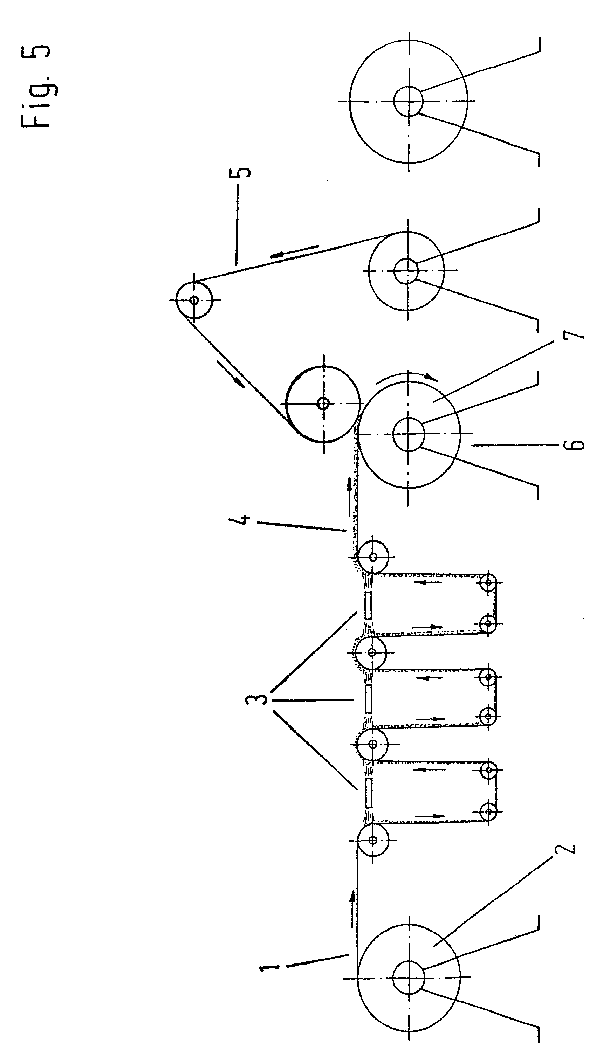

Figur 5 zeigt ein Schema des Herstellungsverfahrens für den dreilagigen Mehrschichtvliesstoff vor dem Hydrophobieren und Bedrucken. Das Trägermaterial 1 wird von der Rolle 2 abgezogen und der elektrostatischen Spinnanlage 3 zugeführt. Nach dem Verlassen der Spinnanlage wird dem mit der Mikrofaserschicht 4 beaufschlagten Trägermaterial der Abdeckvliesstoff 5 zugeführt und mit diesem durch leichtes Anpressen lose verbunden. Das dreilagige Mehrschichtlaminat 6 wird dann über die Rolle 7 abgezogen.

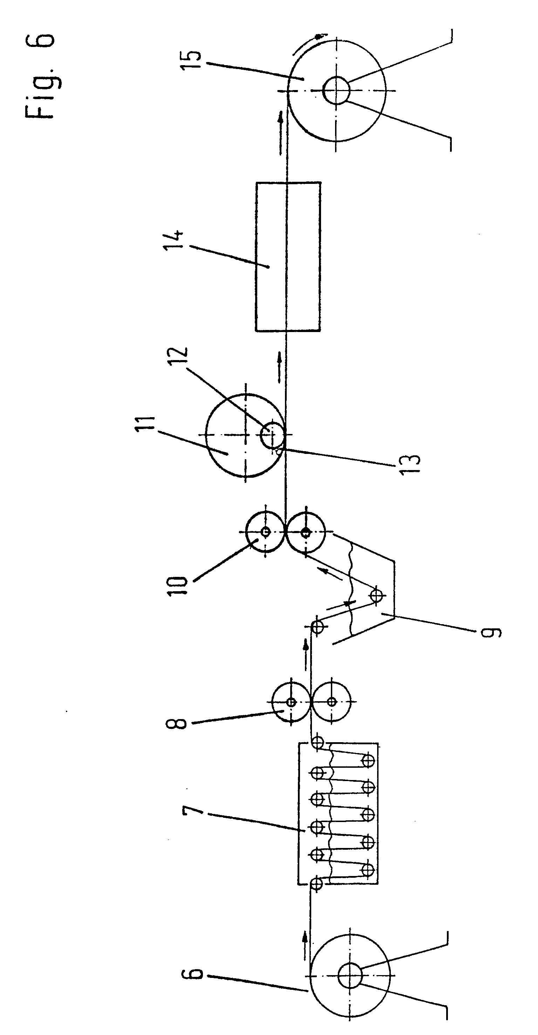

Figur 6 zeigt schematisch die Fertigstellung des Mehrschichtvliesstoffes. Das Dreischichtlaminat 6 wird bei Temperaturen von mehr als 60°C in der Waschmaschine 7 gewaschen. Nach dem Verlassen der Waschmaschine wird es mit Hilfe einer Quetsche 8 abgepreßt und in dem Foulard 9 durch eine Flotte mit dem gewünschten Hydrophobiermittel geführt. Das hydrophob ausgerüstete Mehrschichtgebilde wird dann erneut über die Quetsche 10 entwässert und dann mit Hilfe einer Druckschablone 11, die ein Rollrakel 12 aufweist mitder elastischen hydrophoben Binderpaste 13 bedruckt. Das bedruckte Material wird dem Trockner 14 zugeführt und über die Rolle 15 aufgerollt.

Die nachfolgenden Beispiele erläutern das erfindungsgemäße Verfahren.

Beispiel 1 (dem Stand der Technik entsprechende Nullprobe)

Auf einem Naßvlies mit einem Gewicht von 20 g/m$^2$, bestehend aus 70 % ungemahlenem Zellstoff und 30 % Zellwolle dt 1,7/5 mm, gebunden mit einer hydrophoben emulgatorarmen Polyacrylsäureesterdispersion einer mittelweichen Filmhärte (T300 = ca. -14°C; T300 ist diejenige Temperatur, bei welcher der Torsionsmodul eines luftgetrockneten Filmes einen Wert von 300 kg/cm$^2$ aufweist) und mit einem Bindemittelgehalt von 30 % bezogen auf Vliesstoffgewicht. Anschließend wurden mit dem elektrostatischen Spinnverfahren aus Methylenchloridlösung Mikrofasern aus 93 % Polycarbonat und 7 % Polystyrol aufgesponnen.

Der Mikrofaserauftrag betrug 8 g/m$^2$. Die Faserfeinheit bewegte sich zwischen 1,9 und 9,4 μm, mit einem Mittelwert von 4,5 μm, gemessenan 20 Kapillaren.

Nach dem Bespinnen wurde die zweite Seite der Mikrofaserschicht mit demselben 20 g/m$^2$ schweren Naßvlies abgedeckt, leicht angedrückt und aufgerollt.

Die Wasserdichtheit, gemessen nach DIN 53 886/77, des losen 3-Schicht-Laminates betrug 20 mbar. Die Verbundfestigkeit der drei Schichten zueinander war beinahe 0, wie aus dem folgenden Prüfbericht hervorgeht.

```
Höchstzugkraft längs    33 N/5 cm;   Dehnung bei HZK längs: 7 %
Höchstzugkraft quer     14 N/5 cm;   Dehnung bei HZK quer: 14 %
Spaltfestigkeit         0,4 N*
Luftdurchlässigkeit     240 l/s·m²  bei 0,5 mbar
Dicke                   0,60 mm
```

* Die Spaltung erfolgt innerhalb der Mikroschicht. Ein Verschieben der Schichten erfolgt bereits bei leichtem Andruck mit der Hand.

Eine solche Ware ist insbesondere aufgrund der sehr schwachen Verbundfestigkeit nur für kleinteilige Anwendundungen, wie beispielsweise Einweg-Gesichtsmasken, verwenbar. Für großflächige Anwendungen, wie beispielsweise Schutzbekleidung oder OP-Abdecktücher, ist eine nach Beispiel 1 hergestellte Ware zu steif, zu hart, zu wenig drapierfähig und viel zu schwach im Laminatverbund.

Beispiel 2

Der nach Beispiel 1 hergestellte 48 g/m$^2$ schwere Verbundvliesstoff mit mittiger Mikrofaserschicht, wird im Schritt 2 schematisch dargestellt * . In einer Trommelwaschmaschine wird zuerst bei 60° C und dann bei Raumtemperatur kontinuierlich gewaschen und in einer Quetsche auf eine minimale Feuchte abgepreßt. Anschließend wird ein wässriges Tränkbad, das aus einer 8 %-igen Hydrophobiermittelmischung besteht, durchfahren, d. h. die beschriebene Naß-in-Naß-Tränkung durchgeführt.

Die Naßaufnahme, umgerechnet auf lufttrockenen Vliesstoff, betrug 35 % (= 1,34 g fest/m²). Als Hydrophobiermittel wurde eine zirkonsalzhaltige Paraffinemulsion verwendet. Unmittelbar nach dem Abquetschen der Hydrophobiermitteltränkung, wird die feuchte Ware einseitig mittels einer 10 mesh Rundsiebschablone mit einer Schaumdruckpaste unter Zuhilfenahme eines Magnetdruck-Rakels bedruckt.

```
Rezeptur  der  Mischung:  (mit  einem  Festgehalt  von  40 %)
```

| | Teile fest | Teile flüssig |
|---|---|---|
| Wasser | – | 16,0 |
| anion.Schaummittel | 0,8 | 4,0 |
| grüne Pigmentfarbstoff-zubereitung | 0,5 | 1,0 |
| schwach kationisches Hydrophobiermittel (40 %) | 12,0 | 30,0 |
| 3 % Methylcellulose-Stamm-Ansatz | 0,3 | 10,0 |
| hydrophobe, sehr emulgatorarme Polyacrylat-Dispersion | 100,0 | 222,0 |
| Summe | 113,2 | 283,0 |

Die Methylcellulose hatte einen mittleren Substitutionsgrad von 1,4 - 1,6 und eine Höppler-Viskosität in 2 %iger Lösung von 20.000 CP. Der Auftrag an Druckpaste betrug 10 g/m² Festsubstanz. Die Viskosität der ungeschäumten Mischung betrug 995 CP - Brookfield, gemessen mit Spindel Nr. 4 bei 20 UPM. Die Mischung wurde auf ein Liter-Gewicht von 200 g (Topfgewicht 200 g/Liter) aufgeschäumt.

Nach dem Trocknen war die Ware relativ steif und wurde daher durch Verknüllen mit der Hand mechanisch weich und drapierfähig gemacht.

Folgende Daten wurden gemessen:

Gesamtgewicht: 58 g/m²

Wasserdichtheit: 40,5 mbar

*abgearbeitet

7

Höchstzugkraft längs:    37 N/5cm;  Dehnung bei HZK längs: 14 %

Höchstzugkraft quer:    20 N/5 cm,  "        "     "    quer:   20 %

Dicke:    0,44 mm

Luftdurchlässigkeit:    120 l/s·m²·bei 0,5 mbar

Wasserdampfdurchlässigkeit:    35 mg/h·cm²

Spaltfestigkeit:    1,6 N *


\* Ein Verschieben der Schichten ist absolut nicht mehr
möglich.


Es ist erstaunlich, bis zu welchem Ausmaß im Beispiel 2 die Wasserdichtheit und teilweise die Dehnungen und die Spaltfestigkeit im Vergleich zur Nullprobe 1 angehoben werden konnten. In Relation zu dieser sehr hohen Wasserdichtheit hat Beispiel 2 eine extrem hohe Luftdurchlässigkeit. Aufgrund dieser Tatsache könnte ein Material, das nach Beispiel 2 hergestellt worden ist, als OP-Abdecktuch eingesetzt werden.

Es ist uns kein Verfahren bekannt, mit dem bei einem derart niedrigen Gewicht von 58 g/m² eine so enorm hohe Wasserdichtheit mit sehr hohen Luftdurchlaß verknüpft ist. Hydrophobe Einweg-OP-Abdecktuch-Vliesstoffe liegen bei Wasserdichtheiten von 17 bis 23 mbar, bei Gewichten von 62 bis 80 g/m² und haben je nach Herstellungsmethode und Gewicht, Luftdurchlaßwerte von ca. 30 bis 250 l/s m² gemessen bei 0,5 mbar Luftdruck.

Beispiel 3: Herstellung des grünen Trägermaterials für die Mikrofaserbespinnung.

Auf ein quergelegtes Flor aus 7 g/m² des Faserverschnittes Polyester dtex 1,7/38 mm x Zellwolle dtex 1,3/40 mm = 70 x 30 wird ein 7 g/m² schwerer Längsflor aus 100 % Zellwolle dtex 1,3/40 mm gelegt. Der 2-schichtige Faserverbund wird mittels Schaumimprägnierung mit Bindemittel verfestigt. Die Kunststoffdispersion besteht aus 70 Teilen fest eines weichen selbstvernetzenden Polyacrylates (Acronal 35 D) und 30 Teilen fest eines Klebrohstoffes (Acronal 80 D).

Der Imprägniermischung wurde ein anionischer Schäumer, ein Netzmittel auf Basis Sulfosuccinat und grüner Pigmentfarbstoff zugegeben. Das Verhältnis von Fasermaterial zu Bindemittel betrug 74 : 26, und das Gewicht des Trägervliesstoffes betrug 19 g/m². Der Vliesstoff hatte einen stark hydrophilen Charakter. Der Trägervliesstoff wurde mit der Polymerlösung des Beispiels 1 elektrostatisch besponnen, nur mit dem Unterschied, daß diesmal die durchschnittliche Faserfeinheit der Mikrofaser 2,8 μm (Streuung von 1,1 bis 7,8 μm) betrug.

Der Auftrag an Mikrofasern betrug 8 g/m². Auf die unabgedeckte Mikrofaserschicht wurde ein teilflächig (24 % Verschweißfläche) verschweißtes, 10 g/m² schweres Polyamid-Spinnvlies mit feinem Fasertiter (ca. 2,0 dtex) gelegt und leicht angedrückt. Anschließend wurde, wie im Beispiel 1 beschrieben, gewaschen, naß-in-naß hydrophobiert, naß-in-naß gedruckt und mit hydrophobiermittelhaltiger Schaumpaste getrocknet. Der Auftrag an Hydrophobiermittel betrug 0,8 g/m² und an Schaumpaste 7 g/m², so daß ein Fertigmaterialgewicht von 44,8 g/m² resultierte.

Die stark netzenden Eigenschaften des Trägervliesstoffes begünstigt beim Waschprozess die Durchnetzung der Mikrofaserschicht.

Folgende Werte wurden ermittelt:

```
Wasserdichtheit:                    57 mbar
Luftdurchlässigkeit:                45 1/s·m² bei 0,5 mbar
Wasserdampfdurchlässigkeit: 26 mg/cm²·h
Höchstzugkraft längs:               116 N/5 cm
Höchstzugkraft quer:                52 N/5 cm


Weiterreißkraft längs:              6,4 N/5 cm
Weiterreißkraft quer:               8,3 N/5 cm
```

Die Wasserdichtheit ist so hoch, daß es nicht möglich ist, selbst bei hoher mechanischer Beanspruchung, (wie Klopfen mit der Faust auf eine auf dem Material ausgebreitete Wasserlache) Wasser durchtreten zu lassen. Die Wasserdampfdurchlässigkeit ist dagegen sehr hoch. Aus diesem Grunde könnte ein, nach Beispiel 3 gefertigtes hydrophobes Material, als drapierfähiges semipereables Laminat mit mikroporöser Mikrofasereinlagerung in der Regenschutzbekleidung eingesetzt werden. Die Atmungsaktivität und die hohe Wasserdampfdurchlässigkeit vermitteln ein angenehmes Tragegefühl ohne Gefahr einer Kondenswasserbildung.

Beispiel 4 Dieses Beispiel unterscheidet sich von Beispiel 3 nur dadurch, daß die Mikrofaserauflage von 8 g/m² auf 2,5 g/m² herabgesetzt wurde.

Das Fertigmaterialgewicht betrug nur 39 g/m².
Folgende Werte wurden ermittelt;

```
Wasserdichtheit:            24 mbar
Luftdurchlässigkeit:       182 1/s·m² bei 0,5 mbar
Höchstzugkraft längs:       98 N/5 cm
Höchstzugkraft quer:        48 N/5 cm
Weiterreißkraft längs:     6,0 N
Weiterreißkraft quer:      8,5 N
Fallkoeffizient:            44 %
```

Die nach Beispiel 4 hergestellte Ware besitzt in Bezug auf ihr sehr niedriges Gewicht eine sehr hohe Wasserdichtheit. Die Drapierfähigkeit (Fallkoeffizient) ist sehr gut und wird begünstigt durch das niedrige Gewicht. Das Material kann als Einweg-OP-Abdeckmaterial bzw. Einweg-OP-Kittelmaterial eingesetzt werden. Mit Verfahren des Standes der Technik sind für die gleiche Anwendung Vliesstoffgewichte von mindestens 72 g/m² notwendig. Die Gewichtseinsparung und damit die Rohstoffeinsparung ist also beachtlich.

**Patentansprüche**

1. Drapierfähiger mikroporöser Mehrschichtvliesstoff für medizinische Anwendungszwecke, der eine Mikrofaserschicht aus hydrophoben Fasern enthält, die beidseitig durch gegebenenfalls bindemittelgebundene Vliesstoffe abgedeckt ist, wobei alle Schichten dauerhaft miteinander verbunden sind, dadurch gekennzeichnet, daß die Mikrofaserschicht aus Fasern mit einem Durchmesser im Bereich von 0,1 bis 10 μm besteht, ein Gewicht innerhalb des Bereichs von 0,5 bis 60 g/m² aufweist und daß die Schichten durch eine musterförmig aufgedruckte hydrophobe Bindemittelpaste derart miteinander verbunden sind, daß die Bindemittelpaste an jeder aufgedruckten Stelle in einem säulenförmigen Steg den gesamten Querschnitt aller Schichten durchdringt.

2. Mehrschichtvliesstoff nach Anspruch 1, dadurch gekennzeichnet, daß die Bindemittelpaste auf jeder Seite des Mehrschichtvliesstoffs in Stäbchenform aufgedruckt ist, wobei die einander zugeordneten Stäbchen sich in einem Winkel überkreuzen und an den Kreuzungsstellen jeweils der den Querschnitt der Schichten durchdringende, säulenförmige Steg ausgebildet ist.

3. Mehrschichtvliesstoff nach Anspruch 2, dadurch gekennzeichnet, daß die überkreuzten Stege in einem Winkel von 90° zueinander versetzt sind.

4. Mehrschichtvliesstoff nach Anspruch 1 bis 3, dadurch gekennzeichnet, daß beide Hüllvliesstoffe wasserabweisend ausgerüstet sind.

5. Mehrschichtvliesstoff nach Anspruch 1 bis 3, dadurch gekennzeichnet, daß wenigstens einer der beiden Hüllvliesstoffe aus hydrophilen Fasern aufgebaut ist.

6. Mehrschichtvliesstoff nach einem der Ansprüche 1 bis 5, dadurch gekennzeichnet, daß er mit einer elastischen hydrophoben Bindemittelpaste bedruckt ist.

7. Verfahren zur Herstellung eines mikroporösen Mehrschichtvliesstoffes nach einem der Ansprüche 1 bis 6, dadurch gekennzeichnet, daß auf einem weichen drapierfähigen Trägervliesstoff von 7 bis 50 g/m² Mikrofasern mit einem Durchmesser im Bereich von 1 - 10 μm in einer Menge von 0,5 bis 60 g/m² aufgetragen werden, und daß die Mikrofaserschicht dann mit einem ebenfalls weichen und drapierfähigen Vliesstofflaminat abgedeckt und das so gebildete dreischichtige Laminat durch Verpressen lose verbunden und in Wasser bei Temperaturen über 60°C gewaschen, auf eine Restfeuchte $N_1$ von höchstens 200 Gew.-%, bezogen auf das Trockengewicht des Laminats, abgequetscht und dann gegebenenfalls durch ein wäßriges, ein Hydrophobiermittel enthaltendes Medium geführt und erneut auf eine Restfeuchte $N_2$ von mindestens 50 Gew.-% über Restfeuchte $N_1$ abgequetscht wird und daß das feuchte Laminat dann ein- oder beidseitig mit einer elastischen hydrophoben Druckpaste musterförmig bedruckt wird, wobei die Druckpaste an den Aufdruckstellen alle Schichten in ihrem gesamten Querschnitt als säulenförmige Stege vollständig durchdringt.

8. Verfahren nach Anspruch 7, dadurch gekennzeichnet, daß die Mikrofasern nach dem Verfahren gemäß DE-PS 29 32 072 aus einer Lösung auf den Trägervliesstoff aufgesponnen werden.

9. Verfahren nach Anspruch 7 bis 8, dadurch gekennzeichnet, daß das dreischichtige Laminat vor dem Drucken mit Hydrophobiermittel getränkt wird.

10. Verfahren nach Anspruch 7 bis 8, dadurch gekennzeichnet, daß das dreischichtige Laminat vor dem Drucken nicht mit Hydrophobiermittel getränkt wird.

11. Verfahren nach Anspruch 7, 8 und 10, dadurch gekennzeichnet, daß zumindest einer der beiden Hüllvliesstoffe vor dem Waschen hydrophob ausgerüstet worden ist.

12. Verfahren nach Anspruch 7, 8 und 10, dadurch gekennzeichnet, daß zumindest einer der beiden Hüllvliesstoffe aus saugfähigen Fasern besteht.

13. Verfahren nach Anspruch 7, 8 und 10 und 12, dadurch gekennzeichnet, daß der Hüllvliesstoff musterartig mit Bindemittel beaufschlagt wird.

14. Verfahren nach Anspruch 7, 8, 10, 11 und 12, dadurch gekennzeichnet, daß zumindest einer der beiden Hüllvliesstoffe zum Abdecken der Mikrofaserschicht mit quellfähigem Bindemittel ganzflächig gebunden ist.

15. Verfahren nach Ansprüchen 9 bis 14, dadurch gekennzeichnet, daß die elastische und hydrophobe Druckpaste einseitig aufgedruckt wird.

**16.** Verfahren nach Anspruch 9 bis 14, dadurch gekennzeichnet, daß die hydrophobe Druckpaste aus elastischem Material besteht und beidseitig in Form stäbchenförmiger Stege derart aufgedruckt wird, daß im Überlappungsbereich der einander zugeordneten Stege die den gesamten Querschnitt des Mehrschichtvliesstoffes durchdringenden Druckpunkte gebildet werden.

**17.** Verfahren nach Anspruch 7 bis 10, dadurch gekennzeichnet, daß die elastische hydrophobe Druckpaste beidseitig derart aufgedruckt wird, daß rechteckige, in einem Winkel zueinander versetzte Druckpunkte entstehen, wobei die Druckpaste den gesamten Querschnitt des Mehrschichtvliesstoffes in Form versetzt aufeinander angeordneter Stäbchen durchdringt.

**18.** Verfahren nach Anspruch 7 bis 17, dadurch gekennzeichnet, daß eine wäßrige Druckpaste verwendet wird, die höchstens 1,3 Gew.-% Emulgatoren, höchstens 15,0 Gew.-% Hydrophobiermittel, max. 1,0 % hochmolekulares Verdickungsmittel, ggfs. Pigmentfarbstoff und auf zu 100 Gew.-% ergänzte Trockenmasse der ausschließlich aus hydrophoben Monomerkomponenten aufgebauten Polymerdispersionen enthält, wobei sich die Gewichtsprozente auf das Gesamtgewicht der Druckpastentrockenmasse bezieht.

**19.** Verfahren nach Anspruch 7 bis 18, dadurch gekennzeichnet, daß eine aufgeschäumte wäßrige Druckpaste verwendet wird.

**20.** Verfahren nach Anspruch 19, dadurch gekennzeichnet, daß der Mehrschichtvliesstoff durch ein solches wäßriges hydrophobiermittelhaltiges Medium geführt wird, das einen Entschäumer, z.B.. auf Silikonbasis, enthält.

## Claims

**1.** A drapable microporous multi-layered nonwoven for medical purposes which contains a microfibre layer of hydrophobic fibres which is covered on both sides by self-or binder-bonded nonwovens, all the layers being permanently joined to one another, characterised in that the microfibre layer comprises fibres having a diameter within the range from 0.1 to 10 $\mu$m and has a weight within the range from 0.5 to 60 g/m$^2$ and in that the layers are bonded to one another by a hydrophobic binder paste emprinted in the form of a pattern in such a way that in every emprinted area the binder paste penetrates the entire cross-section of all the layers in the form of a column-shaped stay.

**2.** A multi-layered nonwoven according to claim 1, characterised in that the binder paste is emprinted on each side of the multi-layered nonwoven in rod form, the coordinated rods crossing one another at an angle and the column-shaped stay which penetrates the cross-section of the layers being formed at each of the crossing points.

**3.** A multi-layered non-woven according to claim 2, characterised in that the crossing stays are mutually offset by an angle of 90°.

**4.** A multi-layered nonwoven according to any one of claims 1 to 3, characterised in that the two envelope nonwovens have been rendered water-repellent with a finish.

**5.** A multi-layered nonwoven according to any one of claims 1 to 3, characterised in that at least one of the two envelope nonwovens is composed of hydrophilic fibres.

**6.** A multi-layered nonwoven according to any one of claims 1 to 5, characterised in that it has been printed with an elastic hydrophobic binder paste.

**7.** A process for manufacturing a microporous multi-layered nonwoven according to any one of claims 1 to 6, characterised in that microfibres having a diameter within the range from 1 - 10 $\mu$m are applied in an amount of from 0.5 to 60 g/m$^2$ atop a soft drapable carrier nonwoven of from 7 to 50 g/m$^2$ and in that the microfibre layer is then covered with a likewise soft and drapable nonwoven laminate and the resulting three-layered laminate is loosely bonded together by pressing and washed in water at temperatures above 60°C, squeezed off to a residual moisture content $N_1$ of not more than 200% by weight, based on the dry weight of the laminate, and then optionally passed through an aqueous

EP 0 178 372 B1

medium containing a water repellent and again squeezed off to a residual moisture content $N_2$ of at least 50% by weight above residual moisture content $N_1$, and in that the moist laminate is then printed on one or both sides with an elastic hydrophobic print paste in the form of a pattern, the print paste completely penetrating all the layers in their entire cross-section in the form of column-shaped stays in the emprinted areas.

8. A process according to claim 7, characterised in that the microfibres are spun from a solution onto the carrier nonwoven by the process described in DE-C-2,932,072.

9. A process according to any one of claims 7 to 8, characterised in that the three-layered laminate is impregnated with a water repellent before printing.

10. A process according to any one of claims 7 to 8, characterised in that the three-layered laminate is not impregnated with a water repellent before printing.

11. A process according to claim 7, 8 or 10, characterised in that at least one of the two envelope nonwovens has been rendered water-repellent with a finish before washing.

12. A process according to claim 7, 8 or 10, characterised in that at least one of the two envelope nonwovens comprises absorbent fibres.

13. A process according to claim 7, 8, 10 or 12, characterised in that the envelope nonwoven is treated with binder in the form of a pattern.

14. A process according to claim 7, 8, 10, 11 or 12, characterised in that at least one of the two envelope nonwovens is uniformly covered with swellable binder to cover the microfibre layer.

15. A process according to any one of claims 9 to 14, characterised in that the elastic and hydrophobic print paste is applied to one side.

16. A process according to any one of claims 9 to 14, characterised in that the hydrophobic print paste comprises an elastic material and is applied to both sides in the form of rod-shaped stays in such a way that the print spots which penetrate the entire cross-section of the multilayered nonwoven are formed in the area of overlap of the associated stays.

17. A process according to any one of claims 7 to 10, characterised in that the elastic hydrophobic print paste is applied to both sides in such a way as to form rectangular print spots mutually offset at an angle, the print paste penetrating the entire cross-section of the multi-layered nonwoven in the form of rods arranged on top of one another with offset.

18. A process according to any one of claims 7 to 17, characterised in that an aqueous print paste is used containing not more than 1.3% by weight of emulsifier, not more than 15.0% by weight of water repellent, not more than 1.0% by weight of high molecular weight thickener, optionally a pigment dye and, made up to 100% by weight, dry matter of polymer dispersions composed exclusively of hydrophobic monomer components, the weight percentages being based on the total weight of the print paste dry matter.

19. A process according to any one of claims 7 to 18, characterised in that a foamed aqueous print paste is used.

20. A process according to claim 19, characterised in that the multilayered nonwoven is passed through such an aqueous water repellent medium containing a defoamer, for example a silicone-based defoamer.

**Revendications**

1. Nappe de fibres multicouche microporeuse pouvant être utilisée comme drap pour applications médicales, qui contient une couche de microfibres de fibres hydrophobes, qui est recouverte sur les

12

deux faces par des nappes de fibres liées, éventuellement, par un liant, tandis que toutes les couches sont assemblées de manière durable les unes aux autres, caractérisée en ce que la couche de microfibres est constituée de fibres ayant un diamètre compris dans la gamme de 0,1 à 10 µm présente un poids compris dans la gamme allant de 0,5 à 60 g/m² et en ce que les couches sont assemblées les unes aux autres par une pâte de liant hydrophobe appliquée suivant un motif, de façon à ce que la pâte de liant pénètre à chaque endroit d'application, sous forme d'une saillie en forme de colonne à travers toute la section de toutes les couches.

2. Nappe de fibres multicouche selon la revendication 1, caractérisée en ce que la pâte de liant est appliquée sur chaque face de la nappe de fibres multicouches, sous forme de bâtonnets, tandis que les bâtonnets correspondants les uns aux autres se croisent suivant un certain angle et qu'il se forme chaque fois, au point de croisement, une saillie en forme de colonne traversant la section des couches.

3. Nappe de fibres selon la revendication 2, caractérisée en ce que les saillies des croisements sont décalées les unes par rapport aux autres suivant un angle de 90°.

4. Nappe de fibres multicouche selon les revendications 1 à 3, caractérisée en ce que les deux nappes de fibres enveloppantes sont rendues hydrophobes.

5. Nappe de fibres selon les revendications 1 à 3, caractérisée en ce qu'au moins l'une des deux nappes de fibres enveloppantes est constituée de fibres hydrophiles.

6. Nappe de fibres multicouche selon l'une des revendications 1 à 5, caractérisée en ce qu'on y applique une pâte de liant hydrophobe élastique.

7. Procédé de fabrication d'une nappe de fibres multicouche microporeuse, selon l'une des revendications 1 à 6, caractérisée en ce qu'on applique sur une nappe de support en fibres molle pouvant être formée en drap, de 7 à 50 g/m² de microfibres ayant un diamètre compris dans la gamme de 1 à 10 µm en quantités de 0,5 à 60 g/m² et que la nappe de microfibres est recouverte ensuite d'un stratifié de nappe de libres également mou et pouvant être formée en drap et que le stratifié à trois couches ainsi formé est assemblé de manière lâche par compression et est lavé dans l'eau à des températures supérieures à 60°C, pour obtenir une humidité résiduelle $N_1$ de 200% en poids au maximum, rapporté au poids sec du stratifié, essoré par compression et amené éventuellement à travers un fluide aqueux contenant un agent hydrophobe et essoré à nouveau par compression afin d'obtenir une humidité résiduelle $N_2$ d'au moins 50% en poids supérieure à l'humidité résiduelle $N_1$ et en ce que le stratifié humide reçoit ensuite une application suivant un motif et sur une ou sur les deux faces au moyen d'une pâte d'impression hydrophobe élastique, tandis que la pâte d'impression pénètre complètement au point d'application toutes les couches sur toute leur section, sous forme de saillie en forme de colonne.

8. Procédé selon la revendication 7, caractérisé en ce que les microfibres sont appliquées par filage hors d'une solution suivant le brevet allemand 29 32 072 sur la nappe de fibres de support.

9. Procédé selon les revendications 7 et 8, caractérisé en ce que le stratifié à trois couches est imprégné par un agent hydrophobe avant l'application.

10. Procédé selon les revendications 7 et 8, caractérisé en ce que le stratifié à trois couches n'est pas imprégné par un agent hydrophobe avant l'application.

11. Procédé selon les revendications 7, 8 et 10, caractérisé en ce qu'au moins l'une des deux nappes de fibres enveloppantes est rendue hydrophobe avant le lavage.

12. Procédé selon les revendications 7, 8 et 10, caractérisé en ce qu'au moins l'une des deux nappes de fibres enveloppantes est constituée de fibres absorbantes.

13. Procédé selon les revendications 7, 8, 10 et 12, caractérisé en ce que la nappe de fibres enveloppante reçoit une application d'un liant suivant un motif.

**14.** Procédé selon les revendications 7, 8, 10, 11 et 12, caractérisé en ce qu'au moins l'une des deux nappes de fibres enveloppantes est liée sur toute sa surface par un liant gonflant afin de recouvrir la couche de microfibres.

**15.** Procédé selon les revendications 9 à 14, caractérisé en ce que la pâte d'application hydrophobe et élastique est appliqué sur un seul côté.

**16.** Procédé selon les revendications 9 à 14, caractérisé en ce que la pâte d'application hydrophobe est constituée d'un matériau élastique et est appliquée sur les deux faces, sous forme de languettes en forme de bâtonnets, de façon à ce qu'au point de recouvrement des bandelettes correspondant l'une à l'autre, il se forme des points d'application traversant toute la section de la nappe de fibres multicouche.

**17.** Procédé selon les revendications 7 à 10, caractérisé en ce que la pâte d'application élastique hydrophobe est appliquée sur les deux faces, de façon à obtenir des points d'application rectangulaires décalés les uns par rapport aux autres suivant un certain angle, tandis que la pâte d'application pénètre à travers toute la section de la nappe de fibres multicouche sous forme de bâtonnets décalés les uns par rapport aux autres.

**18.** Procédé selon les revendications 7 à 17, caractérisé en ce qu'on utilise une pâte d'application aqueuse qui contient au maximum 1,3% en poids d'émulsifiants, au maximum 15% en poids d'agents hydrophobes, au maximum 1% d'agents épaississants à poids moléculaire élevé, éventuellement un pigment coloré, en complétant à 100% en poids la masse sèche avec des dispersions polymères formées exclusivement de constituants monomères hydrophobes, tandis que les % en poids se rapportent au poids total de la masse sèche de la pâte d'application.

**19.** Procédé selon les revendication's 7 à 18, caractérisé en ce que l'on utilise une pâte d'application aqueuse transformée en mousse.

**20.** Procédé selon la revendication 19, caractérisé en ce que la nappe de fibres multicouche est amenée à travers un fluide aqueux contenant un agent hydrophobe qui contient un agent antimousse, par exemple à base de silicone.

Fig. 1

Fig. 1a

Fig. 2

Fig. 3

Fig. 4

Fig. 5

EP 0 178 372 B1

Fig. 6

EP 0 178 372 B1